# FASCICULE DE BREVET EUROPEEN

(11) **EP 2 166 975 B1**
(45) Date de publication et mention de la délivrance du brevet: **22.08.2018**
(21) Numéro de dépôt: 08775097.2
(22) Date de dépôt: 15.07.2008
(51) Int. Cl.: A61B 46/00

(54) **PERFECTIONNEMENTS AUX CHAMPS OPERATOIRES A FENETRE**
VERBESSERUNGEN VON CHIRURGISCHEN TÜCHERN MIT FENSTER
IMPROVEMENTS TO SURGICAL DRAPES WITH WINDOW

(30) Priorité: 16.07.2007 FR 0756518
(43) Date de publication de la demande: 31.03.2010
(73) Titulaire: Vygon, 95440 Ecouen (FR)
(72) Inventeur: CARREZ, Jean-Luc, F-95440 Ecouen (FR); HOCQ, Xavier, F-60580 Coye la Forêt (FR); LESTOQUOY, Patrick, F-59551 Attiches (FR)
(74) Mandataire: Regimbeau
(86) Numéro de dépôt international: PCT/EP2008/059238
(87) Numéro de publication internationale: WO 2009/010509

(56) Documents cités:
- WO-A-99/04721
- WO-A-99/16377
- DE-U1- 8 904 426
- US-A- 5 109 873
- US-A- 5 515 868

## Description

L'invention concerne un champ opératoire imperméable qui comporte une fenêtre au travers de laquelle un opérateur peut accéder à un site opératoire et introduire dans le corps d'un patient un tube ou un fil éventuellement prolongé par une ligne de perfusion ou autre ligne qui doit rester en place alors que le champ doit être retiré.

Pour retirer le champ malgré la présence du tube, du fil ou de la ligne qui traverse la fenêtre, il est connu de l'art antérieur de découper le champ au moyen d'un instrument ou de le déchirer au moment voulu pour le retrait, jusqu'à ouvrir latéralement la fenêtre. Ces opérations compliquent le travail de l'opérateur et constituent un risque pour le tube, le fil ou la ligne.

Pour faciliter ces opérations, il a été proposé de munir le champ d'amorces ou de lignes de découpe ou de déchirement, comme décrit par exemple dans les documents EP 1 009 318 et WO 99/16377.

Le document WO 99/16377 décrit des champs à fenêtres utilisés en ophtalmologie dans lesquels sont constituées des lignes qui se rejoignent sur la fenêtre et qui permettent de déchirer ultérieurement le champ pour l'adapter plus facilement à la forme du visage, et notamment des sourcils et paupières du patient. Ces lignes sont constituées par des perforations ou des indentations ou des affaiblissements du film. En fait, la seule méthode effectivement décrite est la découpe de perforations avec un couteau rotatif.

Le document US 5 109 873 décrit un champ opératoire comprenant une nappe présentant une fenêtre d'accès à un site opératoire. La nappe comporte un seul film comprenant une paire de lignes de faiblesse permettant de séparer le film par rupture de la ligne de pliure.

Le document DE 89 04 426 quant à lui décrit un champ opératoire comprenant une nappe présentant une fenêtre d'accès à un site opératoire. Le champ est représenté avant utilisation, et ne comprend qu'une seule feuille, présentant une ligne de déchirement qui peut comprendre des perforations ou une ligne de faiblesse. Les deux parties de la feuille sont alors séparables le long de la ligne de déchirement par rupture de celle-ci. En position d'utilisation, les deux parties sont séparées le long de la ligne de faiblesse, et se chevauchent. Les parties peuvent être maintenues à l'aide d'un adhésif.

D'autre part, si un champ avec une ligne de pointillés pré-découpés peut permettre la séparation, il n'est pas imperméable autour du site opératoire. Or, il doit l'être pour des questions d'aseptie et d'infections possibles. Les indentations ou un affaiblissement thermique fragilisent le champ et favorisent la formation accidentelle de perforations.

Un but de l'invention est de fournir un champ opératoire avec une fenêtre pour l'opération, champ pouvant être séparé ensuite en deux parties pour l'enlever malgré la présence de tubulures sortant du site opératoire par la fenêtre et connectées à des appareils, tout en assurant l'imperméabilité sans fragiliser le champ et de séparer le champ en deux sans l'aide d'un outil coupant.

A cet effet, il est prévu selon l'invention un champ opératoire comprenant une nappe imperméable présentant une fenêtre d'accès au site opératoire, la nappe comportant deux films imperméables pelables maintenus dans le prolongement l'un de l'autre de façon étanche par une soudure thermique qui permet de séparer les films par pelage, la fenêtre étant partagée entre les deux films.

Ainsi, l'utilisation de films imperméables pelables maintenus dans le prolongement l'un de l'autre de façon étanche par une soudure thermique permet de séparer les films l'un de l'autre de manière simple, à moindre effort, sans l'utilisation d'outils coupants et d'autre part, d'assurer l'étanchéité et donc l'imperméabilité du champ autour, en particulier, du site d'opération.

Avantageusement, mais facultativement, le champ opératoire présente au moins l'une des caractéristiques suivantes :
- les deux films présentent deux bords jointifs maintenus par un ruban pelable imperméable placé sous les bords et auquel les bords sont fixés par la soudure thermique ;
- les deux films présentent deux bords en superposition et la soudure thermique est réalisée entre les bords ;
- les bords sont rectilignes ;
- ladite fenêtre est partagée sensiblement par moitié entre les deux films ;
- les films sont rectangulaires ;
- le champ comprend des moyens absorbants autour de la fenêtre fendus pour un pelage latéral ;
- les moyens absorbants comprennent deux bandes absorbantes complémentaires pour former un cadre autour de la fenêtre et se chevauchant, les deux bandes étant collées au champ sauf à des endroits où les deux bandes se chevauchent de sorte qu'elles restent libres à ces endroits ;
- les bandes absorbantes sont conformées en « U » ;
- le champ comporte plusieurs fenêtres superposées de tailles décroissantes réalisées dans des masques successifs pelables qui sont fendus pour un pelage latéral ;
- les films comportent deux feuilles en polyéthylène dont l'une est en polyéthylène à très basse densité.

D'autres caractéristiques et avantages de l'invention apparaîtront lors de la description ci-après, en référence aux figures jointes :
- la figure 1 est une vue de la face de dessous d'un champ opératoire;
- la figure 2 est une vue d'un mode de réalisation d'un champ opératoire conforme à l'invention;
- la figure 3 est une vue du champ de la figure 2 après application d'un adhésif ;
- la figure 4 est une vue de la face de dessus du champ après mise en place de bandes absorbantes ;
- la figure 5 est une vue d'un champ à plusieurs fenêtres superposées ;
- la figure 6 est une vue de la face d'une variante de réalisation d'un champ opératoire ; et
- la figure 7 est une vue d'une variante de réalisation d'un champ opératoire conforme à l'invention.

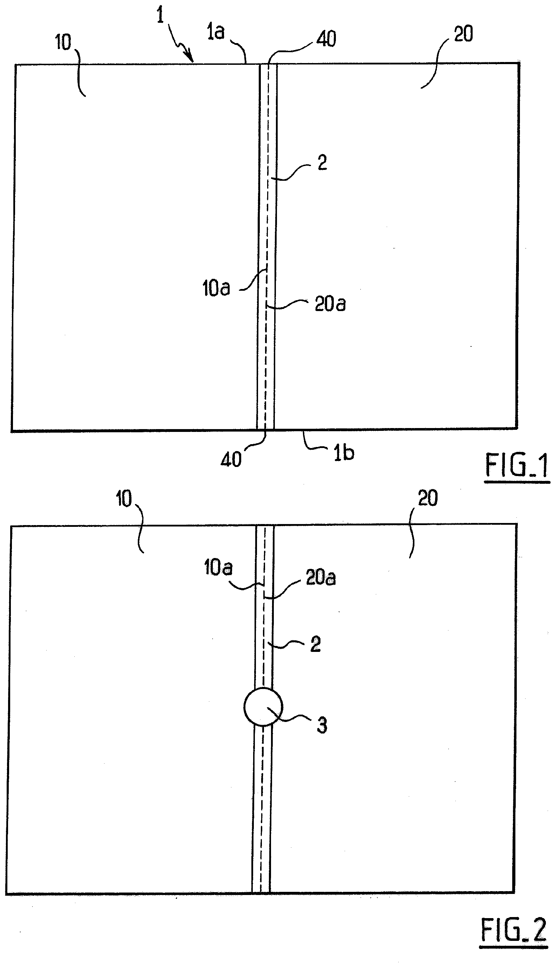

En référence aux figures 1 à 3, nous allons décrire un premier mode de réalisation d'un champ (1) selon l'invention. Le champ (1) comprend deux films pelables imperméables (10, 20) rectangulaires, par exemple de 100 cm x 70 cm environ, dont deux bords (10a, 20a) sont jointifs. Sous ces bords, les films sont fixés à un ruban pelable imperméable (2), par exemple de 5 à 10 cm de largeur environ, par une soudure thermique permettant de détacher ultérieurement les films de la bande par pelage. Nous entendons par film (ruban) pelable un film (ruban) formé de deux feuilles en polyéthylène superposées dont l'une est en polyéthylène très basse densité. La feuille en polyéthylène très basse densité est fine en épaisseur, alors que l'autre feuille en polyéthylène est épaisse. La soudure thermique est réalisée alors que la feuille en polyéthylène très basse densité des films est en contact avec la feuille en polyéthylène du ruban ou inversement. En variante, la soudure thermique est réalisée alors que la feuille en polyéthylène très basse densité des films est en contact avec la feuille en polyéthylène très basse densité du ruban. La soudure thermique consiste en la fusion locale de ces feuilles en polyéthylène très basse densité. En effet, lors de cette soudure thermique, la feuille en polyéthylène très basse densité d'épaisseur fine présente une température de fusion plus basse que celle de la feuille en polyéthylène épaisse qui, elle, ne fond pas lors de cette soudure.

Il est à noter qu'ainsi, lors de la soudure thermique, les feuilles en polyéthylène très basse densité adhèrent entre elles et, lors d'un pelage, la résistance mécanique au déchirement de ces deux feuilles est très faible.

Dans le champ (1) est pratiqué un trou formant une fenêtre (3) partagée entre les deux films, par exemple en deux parties sensiblement égales. Cette fenêtre a toutes formes et toutes dimensions voulues, par exemple circulaire (comme illustré à la figure 2) ou ovale. De l'adhésif (4) est appliqué autour du trou sur la face de dessous du champ et cet adhésif, comme le trou, est protégé provisoirement par un papier siliconé (5) qui sera retiré pour la fixation du champ autour du site opératoire.

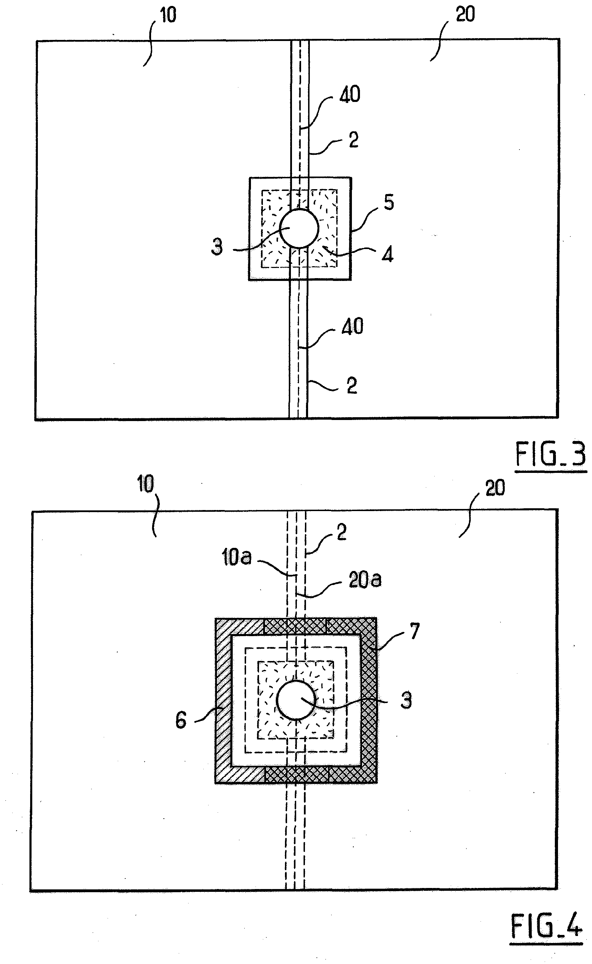

Sur la face de dessus du champ, comme illustré à la figure 4, sont fixées adhésivement autour du trou (3) deux bandes en « U » (6, 7) en matériau absorbant, par exemple en non tissé. Les deux bandes sont placées en opposition de sorte que leurs ailes se chevauchent sur une ligne de coupure (40) et ne soient fixées à l'endroit du chevauchement ni au champ, ni entre elles. Par exemple, les bandes en « U » constituent un carré intérieur d'environ 40 cm de côté et un carré extérieur d'environ 50 à 60 cm de côté. A l'intérieur du carré d'environ 40 cm, tous les matériaux hors papier siliconé sont de préférence transparents.

En variante, le champ comporte, de façon connue en soi, plusieurs fenêtres superposées dont la taille est décroissante depuis la fenêtre initiale du champ jusqu'à la fenêtre située le plus en dessus.

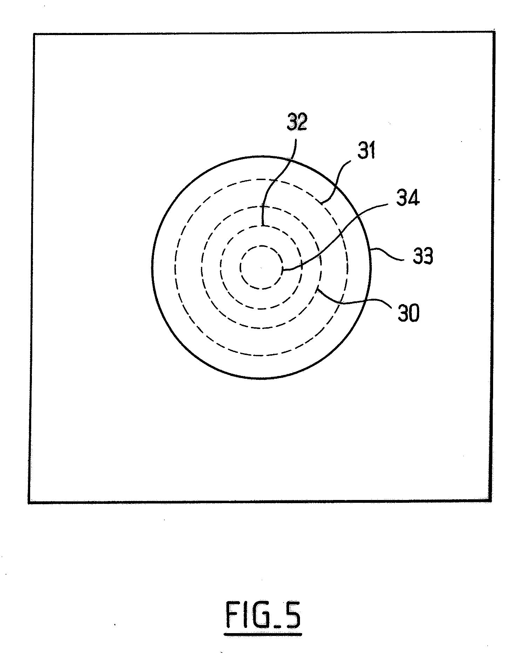

La figure 5 représente un champ qui comporte une fenêtre initiale (30), par exemple d'environ 15 cm de diamètre, recouverte par un premier masque adhésif pelable (31) comportant lui-même une fenêtre (32), par exemple d'environ 10 cm de diamètre, laquelle est recouverte par un deuxième masque adhésif pelable (33) pourvu d'une fenêtre (34) par exemple d'environ 5 cm de diamètre. Les masques sont fendus pour permettre un pelage latéral.

Nous allons maintenant décrire l'utilisation d'un tel champ opératoire. Pour la pose du champ, le papier siliconé est enlevé. Le champ est ensuite appliqué sur la peau du patient. Pour cela, le carré transparent permet de voir à travers le site opératoire. L'opération est ensuite réalisée et des lignes permanentes (perfusion, mesure de pression artérielle et autres) sont mises en place. Ensuite, l'un des films est séparé de la bande en pelant le film latéralement jusqu'au trou du site opératoire. Puis, le champ est tiré latéralement autour des lignes, les bandes en « U » s'écartant aussi sans offrir de résistance autour des lignes installées. Le champ est ensuite décollé de la peau du patient avant de le tirer latéralement.

Il est à noter que les bandes en « U » servent à absorber les liquides lors de l'opération et que le carré de non tissé peut être orienté de manière à éviter d'avoir en position basse, gravitationnellement parlant, du champ les côtés où les bandes se chevauchent.

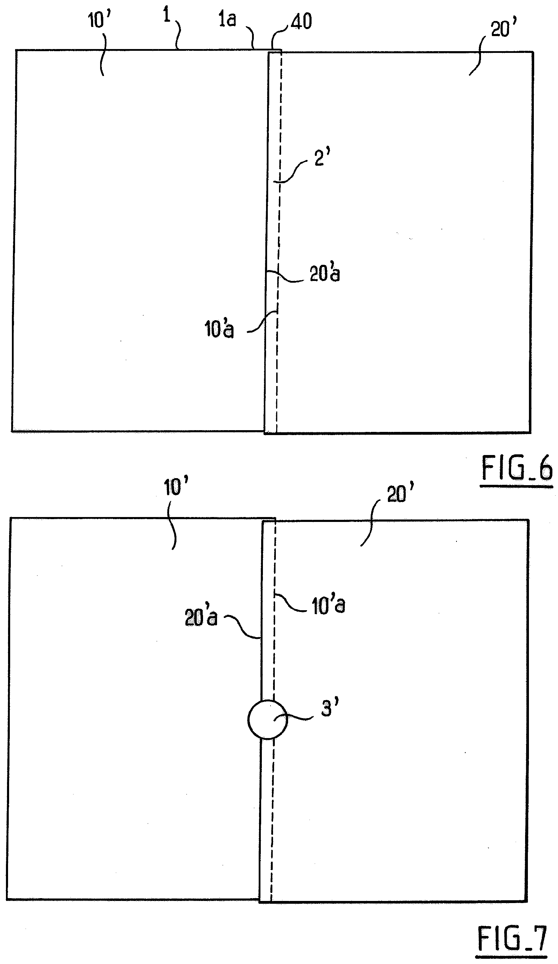

En référence aux figures 6 et 7, nous allons décrire une variante de réalisation d'un champ selon l'invention. Dans cette variante de réalisation, les deux films (10', 20') du champ présentent deux bords (10'a, 20'a) en superposition et une soudure thermique (2') permettant le pelage est réalisée entre les bords ainsi superposés. Le recouvrement des deux films est, par exemple, d'une largeur de 5 cm environ. Lors de ce recouvrement, la feuille en polyéthylène très basse densité de l'un (20') des films est en contact sur ce recouvrement avec la feuille en polyéthylène de l'autre (10') des films. En variante, lors de ce recouvrement, la feuille en polyéthylène très basse densité de l'un (20') des films est en contact sur ce recouvrement avec la feuille en polyéthylène très basse densité de l'autre (10') des films. La soudure thermique consiste en la fusion, au niveau du recouvrement, des feuilles en polyéthylène très basse densité. Un trou (3') est réalisé comme précédemment est réalisé le trou (3).

L'invention n'est pas limitée aux réalisations qui ont été décrites.

## Revendications

1. Champ opératoire comprenant une nappe imperméable présentant une fenêtre (3 ; 3') d'accès à un site opératoire, **caractérisé en ce que** la nappe comporte deux films imperméables pelables (10, 20 ; 10', 20'), maintenus dans le prolongement l'un de l'autre de façon étanche par une soudure thermique qui permet de séparer les films par pelage et **en ce que** la fenêtre (3 ; 3') est partagée entre les deux films (10,20; 10',20').

2. Champ opératoire selon la revendication 1, **caractérisé en ce que** les deux films (10,20; 10',20') présentent deux plans jointifs (10a, 20a) maintenus par un ruban pelable imperméable (2) placé sous les bords et auquel les bords sont fixés par soudure thermique.

3. Champ opératoire selon la revendication 1, **caractérisé en ce que** les deux films (10', 20') présentent deux bords (10'a, 20'a) en superposition et **en ce que** la soudure thermique (2') est réalisée entre lesdits bords (10'a, 20'a).

4. Champ opératoire selon la revendication 1 ou 2, **caractérisé en ce que** les bords (10a, 20a) sont rectilignes.

5. Champ opératoire selon l'une des revendications 1 à 4, **caractérisé en ce que** la fenêtre (3; 3') est partagée sensiblement par moitié entre les deux films (10,20; 10',20').

6. Champ opératoire selon l'une des revendications 1 à 5, **caractérisé en ce que** les films (10,20; 10',20'). sont rectangulaires.

7. Champ opératoire selon l'une des revendications 1 à 6, **caractérisé en ce qu'**il comprend des moyens absorbants autour de la fenêtre (3; 3') qui sont fendus pour un pelage latéral.

8. Champ opératoire selon la revendication 7, **caractérisé en ce que** les moyens absorbants comprennent deux bandes absorbantes (6, 7) complémentaires pour former un cadre autour de la fenêtre (3) et se chevauchant, les deux bandes absorbantes (6, 7) étant collées au champ sauf à des endroits où les deux bandes absorbantes (6, 7) se chevauchent de sorte qu'elles restent libres à ces endroits.

9. Champ opératoire selon la revendication 8, **caractérisé en ce que** les bandes absorbantes (6, 7) sont conformées en « U ».

10. Champ opératoire l'une des revendications 1 à 9, **caractérisé en ce qu'**il comporte plusieurs fenêtres (30, 32, 34) superposées de taille décroissante réalisées dans des masques successifs (31, 32) pelables qui sont fendus pour un pelage latéral.

11. Champ opératoire l'une des revendications 1 à 10, **caractérisé en ce que** les films (10,20; 10',20'). comportent deux feuilles en polyéthylène dont l'une est en polyéthylène très basse densité.

## Patentansprüche

1. Operationsfeld umfassend ein undurchlässiges Tuch, das ein Fenster (3; 3') für den Zugang zu einem Operationsort aufweist, **dadurch gekennzeichnet, dass** das Tuch zwei undurchlässige abziehbare Folien (10, 20; 10', 20') aufweist, die in der jeweiligen Verlängerung durch eine thermische Schweißung dicht gehalten werden, die es ermöglicht, die Folien durch Abziehen zu trennen, und dadurch, dass das Fenster (3; 3') zwischen den beiden Folien (10, 20; 10', 20') geteilt ist.

2. Operationsfeld nach Anspruch 1, **dadurch gekennzeichnet, dass** die beiden Folien (10, 20; 10', 20') zwei anstoßende Ebenen (10a, 20a) aufweisen, die von einem abziehbaren undurchlässigen Band (2) gehalten werden, das unter den Kanten angeordnet ist und an dem die Kanten durch thermisches Schweißen befestigt sind.

3. Operationsfeld nach Anspruch 1, **dadurch gekennzeichnet, dass** die beiden Folien (10', 20') zwei übereinanderliegende Kanten (10'a, 20'a) aufweisen und dadurch, dass das thermische Schweißen (2') zwischen diesen Kanten (10'a, 20'a) durchgeführt wird.

4. Operationsfeld nach Anspruch 1 oder 2, **dadurch gekennzeichnet, dass** die Kanten (10a, 20a) geradlinig sind.

5. Operationsfeld nach einem der Ansprüche 1 bis 4, **dadurch gekennzeichnet, dass** das Fenster (3; 3') im Wesentlichen jeweils zur Hälfte auf die beiden Folien (10, 20; 10', 20') aufgeteilt ist.

6. Operationsfeld nach einem der Ansprüche 1 bis 5, **dadurch gekennzeichnet, dass** die Folien (10, 20; 10', 20') rechteckig sind.

7. Operationsfeld nach einem der Ansprüche 1 bis 6, **dadurch gekennzeichnet, dass** es um das Fenster (3; 3') herum absorbierende Mittel umfasst, die zum seitlichen Abziehen geschlitzt sind.

8. Operationsfeld nach Anspruch 7, **dadurch gekennzeichnet, dass** die absorbierenden Mittel zwei komplementäre absorbierende Streifen (6, 7) umfassen, um einen Rahmen um das Fenster (3) herum und sich überlappend zu bilden, wobei die beiden absorbierenden Streifen (6, 7) an das Feld geklebt sind außer an den Stellen, an denen sich die beiden absorbierenden Streifen (6, 7) überlappen, so dass sie an diesen Stellen frei bleiben.

9. Operationsfeld nach Anspruch 8, **dadurch gekennzeichnet, dass** die absorbierenden Streifen (6, 7) U-förmig sind.

10. Operationsfeld nach einem der Ansprüche 1 bis 9, **dadurch gekennzeichnet, dass** es mehrere übereinanderliegende Fenster (30, 32, 34) von abnehmender Größe umfasst, die in aufeinanderfolgenden abziehbaren Masken (31, 32) ausgeführt und zum seitlichen Abziehen geschlitzt sind.

11. Operationsfeld nach einem der Ansprüche 1 bis 10, **dadurch gekennzeichnet, dass** die Folien (10, 20; 10', 20') aus zwei Polyethylenfolien bestehen, von denen eine aus Polyethylen sehr niedriger Dichte besteht.

## Claims

1. An operating drape comprising an impermeable cloth having a window (3; 3') for accessing an operating site, **characterized in that** the cloth includes two peelable impermeable films (10, 20; 10', 20'), maintained in the extension of each other in a sealed manner by means of a heat weld making it possible to separate the films by peeling and **in that** the window (3; 3') is shared between both films (10, 20; 10', 20').

2. The operating drape according to claim 1, **characterized in that** the two films (10, 20; 10', 20') have two joined planes (10a, 20a) maintained by an impermeable peelable strip (2) placed under the edges and to which the edges are attached by heat welding.

3. The operating drape according to claim 1, **characterized in that** the two films (10', 20') have two superposed edges (10'a, 20'a) and **in that** the heat weld (2') is achieved between said edges (10'a, 20'a).

4. The operating drape according to claim 1 or 2, **characterized in that** the edges (10a, 20a) are rectilinear.

5. The operating drape according to one of claims 1 to 4, **characterized in that** the window (3; 3') is substantially half-shared between both films (10, 20; 10', 20').

6. The operating drape according to one of claims 1 to 5, **characterized in that** the films (10, 20; 10', 20') are rectangular.

7. The operating drape according to one of claims 1 to 6, **characterized in that** it comprises absorbent means around the window (3; 3') which are slit for lateral peeling.

8. The operating drape according to claim 7, **characterized in that** the absorbent means comprise two mating absorbent strips (6, 7) for forming a frame around the window (3) and overlapping, both absorbent strips (6, 7) being adhesively bonded to the drape except in locations where both absorbent strips (6, 7) overlap so that they remain free in these locations.

9. The operating drape according to claim 8, **characterized in that** the absorbent strips (6, 7) are U-shaped.

10. The operating drape according to one of claims 1 to 9, **characterized in that** it includes several superposed windows (30, 32, 34) with decreasing size made in successive peelable masks (31, 32) which are slit for lateral peeling.

11. The operating drape according to one of claims 1 to 10, **characterized in that** the films (10, 20; 10', 20') include two polyethylene sheets, one of which is in ultra low density polyethylene.
